**Europäisches Patentamt**

(19) **European Patent Office**

**Office européen des brevets**

(11) Publication number : **0 323 818 B1**

## (12) EUROPEAN PATENT SPECIFICATION

(45) Date of publication of patent specification :
04.03.92 Bulletin 92/10

(51) Int. Cl.⁵ : **A61F 2/04, A61B 19/00**

(21) Application number : **88830570.3**

(22) Date of filing : **29.12.88**

(54) **Autostatic urethral endoprosthesis.**

(30) Priority : **30.12.87 IT 2956387 U**

(43) Date of publication of application :
**12.07.89 Bulletin 89/28**

(45) Publication of the grant of the patent :
**04.03.92 Bulletin 92/10**

(84) Designated Contracting States :
**CH DE ES FR GB IT LI**

(56) References cited :
**WO-A-83/03752**
**CH-A- 661 665**
**DE-A- 3 339 179**
**DE-B- 2 528 273**
**FR-A- 2 310 775**
**GB-A- 1 518 654**
**US-A- 3 592 197**

(56) References cited :
**US-A- 3 868 956**
**US-A- 4 592 341**
**A.S.A.I.O. TRANSACTIONS, vol. 33, no. 3, September 1987, pages 480-481, Hagerstown, MD, US; D. CHOPIN et al.: "Autostatic endourethral prosthesis for long-term modeling-replacement of the male urethra"**

(73) Proprietor : **FINA, Ernesto**
**Piazza Leonardo, 10, Viale Villa Maio**
**I-80129 Napoli (IT)**

(72) Inventor : **FINA, Ernesto**
**Piazza Leonardo, 10, Viale Villa Maio**
**I-80129 Napoli (IT)**

(74) Representative : **Bazzichelli, Alfredo et al**
**c/o Società Italiana Brevetti S.p.A. Piazza di Pietra, 39**
**I-00186 Roma (IT)**

EP 0 323 818 B1

Jouve, 18, rue Saint-Denis, 75001 PARIS

# Description

The present invention refers to a urethral autostatic endoprosthesis as specified in the preambule of Claim 1, i.e. an endoprosthesis provided with means capable of self-connection in bladder and prostatic urethra for transurethrally draining vesical urines. Such an endoprosthesis is known from US-A-3592197.

As is known, the problem of transurethrally draining vesical urines has been solved since the first steps of the urological practice by means of tubular instruments. The evolution of the relevant technology has caused such instruments, called catheters, to undergo modifications and improvements to meet the requirements of the more and more sophisticated techniques. Such modifications have concerned the shape of the catheter's proximal end (the one to be inserted into the bladder), the gauge and first of all the material to be used in order to progressively reduce the traumatic effects of catheters and improve their compatibility with urines.

The first catheters, which are still widely employed, were the non-autostatic ones, i.e. catheters without means capable of self-connection in the bladder. In the case of a long permanence in the body, these catheters had to be secured to external genitals with mechanical means (plasters, threads and so on).

Other vesical catheters, which appeared later, in cluded the ones which are self connectable in the bladder. These devices comprise either distorted or expanded ends (Malecot type), see also US-A-3 592 197, or protuberances on the lateral cylindrical surface of the catheter in the form of "bards" (see US-A-4 592 341 and A-S-A-I-O Transactions, Vol. 33, no.3, pages 480-481) or irregularities (see DE-B-2 528 273) or balloons to be inflated from the outside which are present at a short distance from the proximal (vesical) end of the catheter.

Irrespective of the features of the available catheters, the catheter's function is to allow the vesical urines to be drained notwithstanding an obstacle in the urethra or pathological reasons prevent the bladder from being naturally emptied.

Taking into account only urinary inability caused by obstacles in the cervical prosthatic area (where the device according to the present invention can be applied), one can say that such obstacles depend on either deseases which have the effect of occluding the cervical cervix or, in a greater extent, increase of the prosthatic gland, the above increase being either of benign nature (fibromioadenomatous hyperplasia) or of malignant nature (carcinoma).

The above deseases can be treated by surgical operations (in the open air or endoscopically) which result in remotion of the obstacle and in restoration of the normal urinary function.

In the case where the surgical operation is impossible in view of either the excessive risks of narcosis or the patient's explicit refusal, the only possible treatment is the application of an externally anchored catheter.

Such a treatment solves a big problem, but in time creates other problems, the gravity of which is not negligible. First of all it causes the patient to experience psychological troubles deriving from the continuous presence of a foreign body which comes out from the genital organ and upon which depends a function as important as the urinary one (sense of disability and frustration). Moreover, the problems related to the catheter's maintainance are not less serious than the psychological ones (patency control and periodical substitution). Furthermore, the above treatment can be the cause of bacterial infection in the bladder which is hard to control, and which, in some cases, reaches the kidneys with a serious deterioration of their function.

Notwithstanding the high performance of the above instruments, especially as regards tollerance and technical quality, said problems remain substantially unsolved.

Over the above state of the art, the urethral autostatic endoprosthesis represents an unexpected means of overcoming said drawbacks. In fact its use prevents psychological or physical troubles, permits an almost natural urinary act, and prevents, as far as possible, the negative effects of bacterial infection of the bladder.

The urethral autostatic endoprosthesis according to the invention, which can be self-connected to the prosthatic urethra for transurethrally draining vesical urines, comprises the following parts: a hollow tubular structure, made of biocompatible substantially non-deformable material, the above structure optionally being provided with longitudinally extending openings onto the lateral surface; a first pair of tongues (proximal tongues) made of biocompatible, flexible, non-traumatic material, onto an end of the above tubular structure, said tongues being substantially flared prolongations of portions of said end and being substantially mutually opposite; a second pair of tongues (distal tongues), made of biocompatible, flexible, non-traumatic material, onto the other end of the above tubular structure, said tongues being substantially flared prolongations of portions of said end, substantially mutually opposite and smaller than the tongues of the above first pair; and, optionally, a biocompatible soft coating for making the whole prosthesis non-traumatic.

Naturally the length and gauge of the hollow tubular structure can vary in a wide range.

The present invention is not limited to the urethral autostatic endoprosthesis, but also covers a specific device for its application.

The device for applying the urethral autostatic endoprosthesis according to the present invention comprises the following parts: a tube-shaped guide, as long as a conventional jacket of an urethroscope - with an end (proximal end) having a truncated bevelled tip and the other end (distal end) having a truncated tip - provided with longitudinally extending openings starting from the distal end; and a pushing mandrel - consisting of a stem, optionally graduated, made of a stiff plastic material - which has an end with the form of a bevelled ogive coinciding with the truncated bevelled proximal end of said guide and with the other end perpendicularly equipped with a round plate having a diameter capable of allowing a tight insertion into said guide, two holes crossing said ogive-shaped end.

The objects, features and advantages of the invention will be further clarified by means of the annexed figures.

Figure 1 shows a perspective view of the urethral autostatic endoprosthesis according to the invention.

Figure 2 shows a side view of the guide of the device for applying the urethral autostatic endoprosthesis according to the invention.

Figure 3 shows a perspective view of the pushing mandrel of the device for applying the urethral autostatic endoprosthesis according to the invention.

Figure 4 represents a perspective view where the device for applying the urethral autostatic endoprosthesis according to the invention is shown with the mandrel inserted into the guide.

Figure 5 represents a perspective view where the device according to the invention is shown when pushing the urethral autostatic endoprosthesis.

Figures 6 and 7 schematically show the prosthatic gland with obstruction of prosthatic urethra respectively before and after the insertion of the urethral autostatic endoprosthesis according to the invention.

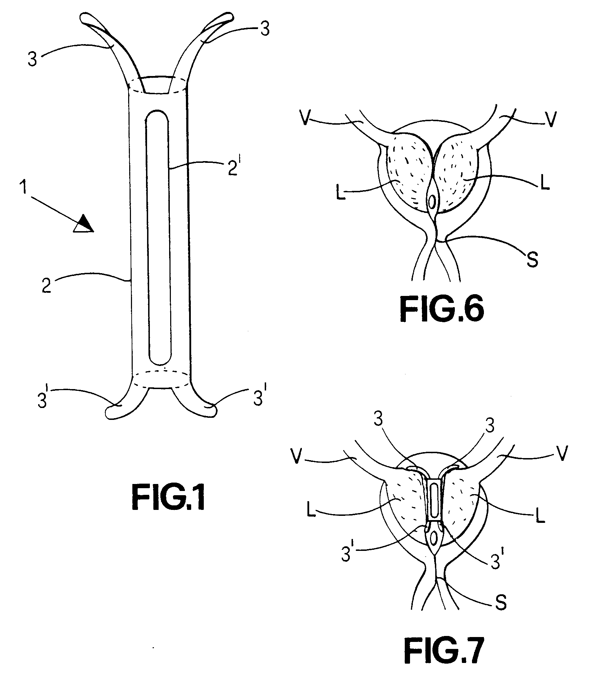

With reference to figure 1, the urethral autostatic endoprosthesis 1 comprises a hollow tubular structure 2 with longitudinal openings 2' onto its lateral surface. Said tubular structure 2 bears on each of its ends a pair of tongues 3 and 3' for assuring an autostatic fitting.

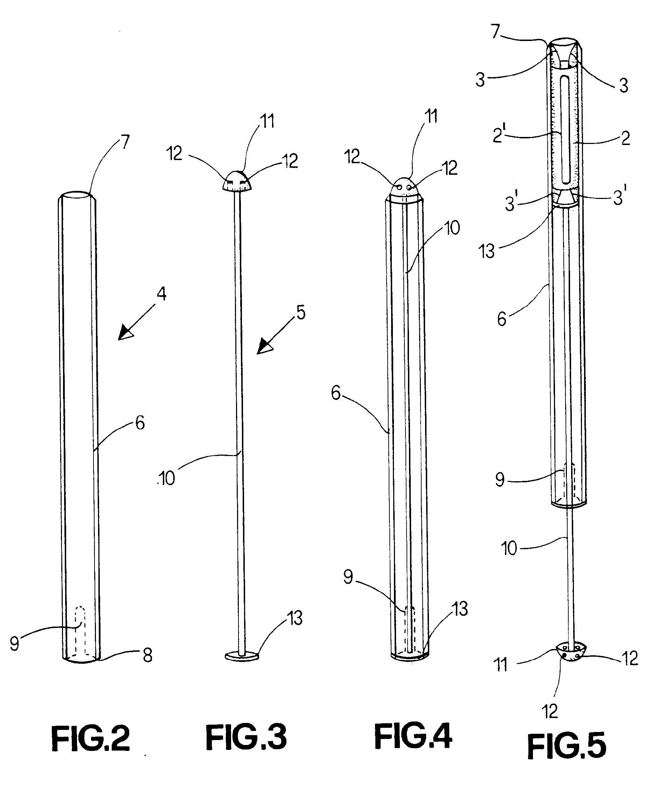

With reference to figures 2 to 5, the device consists of two parts: a guide 4 and a pushing mandrel 5. The guide is a cylindrical tube 6 - made of stiff plastic material with proximal end 7 (with respect to the patient) having a truncated bevelled tip and distal end 8 having a truncated tip - as long as a conventional jacket of an urethroscope and equipped with longitudinally extending openings starting from the distal end. The other component of the device is the pushing mandrel 5 which has the function of rendering non-traumatic the guide's insertion, as well as the function of pushing the prosthesis, when inserted into the guide, up to the proper seat. The mandrel 5 consists of a cylindrical stem 10 made of stiff plastic material having an end with the form of a bevelled ogive 11 which perfectly coincides with the truncated bevelled proximal end 7 of the guide 4. This ogive-shaped head is crossed by two holes 12. The other end of the mandrel 5 is perpendicularly equipped with a round plate 13 having a diameter capable of allowing a tight insertion into the guide when the device is ready for use (guide with the mandrel inside).

This endoprosthesis should be applied in the prosthatic urethra in such a way that the proximal end, just appearing in the bladder V, rests on the vesical cervix and is held tight thereon by the tongues 3 (the bigger pair). The distal end of the prosthesis should just arrive at the maximum prominence of the hypertrophic prostatic lobes L so that the tongues 3' (the smaller pair) can run along the lower edge of the prostatic lobes, between the latter and the external voluntary sphincter S.

In order to put the prosthesis in situ, either a conventional jacket of a tip-truncated urethroscope or the device according to the invention can be used. In the case of a prosthesis being applied for the first time, it is possible to use the disclosed device even in the patient's home. On the contrary, when a prosthesis is to be replaced, or whenever it is important to check either the condition of the prosthatic lobes or the condition of the prosthesis, it is necessary to use to urethroscope.

The method for inserting the prosthesis by means of the device according to the invention is as follows. The guide 4 is inserted, with the mandrel 5 inside, in the external urinary meatus and is pushed along the previously lubricated urethra until said guide is engaged in the prosthatic urethra after having overcome the voluntary sphincter. At this point it is necessary to proceed very carefully and slowly. As soon as the guide 4 arrives at the bladder (previously held full of urine), urine crosses the holes 12 in the ogive 11 of the mandrel, enters the guide 4 and is discharged outside via the openings 9 at the distal end 8 of the guide 4. As soon as the urine is discharged, the guide should be stopped in that position and the mandrel 5 should be taken out. Then, starting from the tongues 3 held aligned with the corresponding portion of the lateral surface of the hollow tubular structure 2, the autostatic prosthesis is introduced inside the guide 4. After having completely introduced the prosthesis inside the guide, the mandrel 5 with the plated end 13 towards the bladder, is inserted and the prosthesis is pushed until the proximal tongues come out of the end of the guide and open onto the vesical cervix. In this position the distal end 8 of the guide 4 collimates with one of the graduations in the stem of the mandrel. Now the device for applying the prosthesis can be taken out, since the prosthesis is in the right position.

The application with the urethroscope is simpler, since the right position of the device can be checked by sight. As soon as the urethroscope is in correspondence with said cervix, the optical system is taken out and the same procedure as described hereinbefore is followed by using the urethroscope's jacket instead of the guide 4. After insertion the right position of the prosthesis can be endoscopically checked with reference to the position of distal end and distal tongues.

Before the application it is very important to estimate how long the prosthesis to be installed in the patient under treatment should be. The above result can be obtained by subjecting the patient to an ecotomographic examination which in turn gives the longitudinal diameter of the gland, i.e. the distance between the base and the apex of the same. The prosthesis length from the proximal ring (at the base of the bigger tongues) to the apex of the smaller tongues should not exceed the longitudinal diameter of the gland.

The removal of the prosthesis should be carried out by means of the urethroscope by grasping the distal tongues with suitable endoscopic forceps. A weak traction is sufficient for the prosthesis to reach the pene urethra and to be taken out.

The urethral autostatic endoprosthesis according to the present invention represents an alternative to the vesical non-surgically applied catheters over which it exhibits the advantages mentioned hereinbefore. Besides the practical and psychological benefits, the broadest almost unexpected advantage of the invention is the possibility of obtaining an almost apparently normal urination. However, in order to enjoy a satisfactory urinary function, it is likely that the patient will spend a period of time in accustoming himself to the prosthesis and first of all in training himself to use only the voluntary sphincter in order to control urination.

In fact, as is known, in the normal subject the continence is assured by the tonic contraction of the non-voluntary sphincter which has its seat connected with the vesical cervix. On the contrary, in the patient with the urethral endoprosthesis, the non-voluntary sphincter cannot work, since it is held open by the prosthesis. Therefore, only the voluntary sphincter will retain its function and will prevent incontinence. These patients experience that which is experienced by patients subjected to surgical or endoscopic removal of prosthatic adenoma which results in ablation of the non-voluntary sphincter. In such a case it is possible, after a transitory period, to recover a completely normal function.

## Claims

1. A urethral autostatic endoprosthesis, which can be self-connected to the prosthatic urethra for transurethrally draining vesical urines, comprising a hollow tubular structure (2), made of biocompatible material, a first pair of tongues (3) (proximal tongues) made of biocompatible, flexible, non-traumatic material, onto an end of the above tubular structure (2), said tongues being substantially flared prolongations of portions of said end and being substantially mutually opposite; characterized in that said hollow tubular structure is made of a substantially non-deformable material and is optionally provided with longitudinally extending oppenings (21) onto its lateral surface, and a second pair of tongues (31) (distal tongues), made of biocompatible, flexible, non-traumatic material, are provided onto the other end of the above tubular structure, said tongues being substantially flared prolongations of portions of said end, substantially mutually opposite and smaller than the tongues of the above first pair, and, optionally, a biocompatible soft coating is provided for making the whole prosthesis non-traumatic.

2. A device for applying the urethral autostatic endoprosthesis according to claim 1, comprising the following parts: a tube-shaped guide (4), as long as a conventional jacket of a urethroscope - with an end (7) (proximal end) having a truncated bevelled tip and the other end (8) (distal end) having a truncated tip - provided with longitudinally extending openings (9) starting from the distal end; and a pushing mandrel (5) - consisting of a stem (10), optionally graduated, made of a stiff plastic material - which has an end (11) with the form of a bevelled ogive coinciding with the truncated bevelled proximal end (7) of said guide and with the other end perpendicularly equipped with a round plate (18) having a diameter capable of allowing a tight insertion into said guide (4), two holes (12) crossing said ogive-shaped end (13).

## Patentansprüche

1. Eine selbsttragende Harnröhrenendoprothese, die bei Prostatahypertrophie mit der Harnröhre verbunden werden kann, um den Blasenharn durch die Harnröhre abzuleiten, wobei die Prothese einen aus biologisch verträglichem Material bestehenden hohlen rohrförmigen Körper (2), und ein erstes aus biologisch verträglichem biegsamen nicht traumatischen Material bestehendes Paar Zungen (31) (Nahzungen) an einem Ende des rohrförmigen Körpers (2) aufweist, wobei die Zungen im wesentlichen als ausgeweiterte Fortsetzungen von Endpartien sich im wesentlichen entgegengesetzt erstrecken, dadurch gekennzeichnet, dass der rohrförmige Körper aus einem im wesentlichen nicht verformbaren Material besteht und gegebenenfalls mit Längsöffnungen (21) auf dessen seitlicher Oberfläche versehen ist, und ein zweites aus biologisch verträglichem, biegsamen, nicht traumatischen Material bestehendes Paar Zungen (31) (Distalzungen) auf dem entgegengesetzten Ende des rohrförmigen Körpers angeordnet sind, wobei die Zungen im wesentlichen als ausgeweitete Fortsetzungen von Endpartien und sich im wesentlichen entgegengesetzt erstrecken, und kleiner als die ersten Zungen sind, und auf denen gegebenenfalls eine biologisch verträgliche weiche Beschichtung aufgetragen ist, so dass die gesamte Prothese nicht traumatisch wirken kann.

2. Eine Vorrichtung um die selbsttragende Harnröhrenendoprothese nach Anspruch 1 anzubringen, bestehend aus folgenden Teilen: eine rohrförmige Leitvorrichtung (4) in der Länge der gewöhnlichen Verkleidung eines Harnröhrenspiegels, die mit Längsöffnungen (9), die am Distalende beginnen, versehen ist, wobei ein Ende (7) (Nahende) eine abgeschrägte Spitze aufweist und das andere Ende (Distalende) eine abgebrochene Spitze aufweist, und ein Führungsmandrel (5) - der aus einem aus unbiegsamen Kunststoffmaterial gegebenenfalls graduiertem Stiel besteht, wobei der Mandrel an einem Ende (11) die Form eines schrägen Spitzbogens aufweist, die mit dem abgeschrägten Nahende (7) der Leitvorrichtung übereinstimmt, und am anderen Ende mit einer senkrecht angeordneten runden Platte (13) versehen ist, und die runde Platte (13) einen Durchmesser aufweist, der eine dichte Anpassung in die Leitvorrichtung (4) erlaubt, wobei zwei Löcher (12) durch das spitzbogenförmige Ende (11) angebracht sind.

## Revendications

1. Endoprothèse urétrale autostatique qui peut être connectée directement à l'urètre prostatique pour drainer au-delà de l'urètre les urines véscicales, comprenant une structure tubulaire creuse (2) de matériel biocompatible, une première couple de languettes (3) (languettes proximales) de matériel biocompatible, flexible, non-traumatique sur une extrémité de ladite structure tubulaire (2), lesdites languettes étant substantiellement des prolongements évasés de parties de ladite extrémité et étant substantiellement opposées l'une à l'autre, caractérisée en ce que ladite structure tubulaire creuse est faite d'un matériel substantiellement non déformable et est éventuellement pourvue d'ouvertures (21) étendues longitudinalement sur sa surface latérale; une deuxième paire de languettes (31) (languettes distales) de matériel biocompatible, flexible, non traumatique est placée sur l'autre extrémité de ladite structure tubulaire, lesdites languettes étant substantiellement un prolongement évasé de parties de ladite extrémité, substantiellement opposées l'une à l'autre et plus petites que les languettes de ladite première paire et, éventuellement, un revêtement souple, biocompatible est prévu pour rendre non traumatique l'entière prothèse.

2. Dispositif pour appliquer la prothèse urétrale autostatique selon la revendication 1, comprenant les parties suivantes: un guide (4) à tuyau de la même longueur que le manteau conventionnel d'un urétroscope, avec une extrémité (7) (extrémité proximale) ayant une pointe tranchée bisautée, pourvue d'ouvertures (9) étendues longitudinalement à partir de l'extrémité distale; et un mandrin (5) de poussée, constitué d'une tige (10), éventuellement graduée, de matériel plastique rigide, ayant une extrémité en forme d'ogive bisautée, correspondant à l'extrémité proximale (7) tranchée et bisautée dudit guide et l'autre extrémité pourvue d'une plaque ronde (13) perpendiculaire, d'un diamètre capable de permettre l'insertion hermétique dans le guide (4), deux trous traversant l'extrémité en ogive.

FIG.1

FIG.6

FIG.7

**FIG.2**

**FIG.3**

**FIG.4**

**FIG.5**

7